# EUROPEAN PATENT SPECIFICATION

(11) **EP 3 471 836 B1**
(45) Date of publication and mention of the grant of the patent: **22.05.2024**
(21) Application number: 17732379.7
(22) Date of filing: 20.06.2017
(51) Int. Cl.: A61Q 19/00, A61K 8/99, A61P 17/00, A61K 35/747, C12R 1/25, C12R 1/225, A61K 9/00, C12N 1/20

(54) **DERMATOLOGICAL PREPARATIONS FOR MAINTAINING AND/OR RESTORING HEALTHY SKIN MICROBIOTA**
DERMATOLOGISCHE ZUBEREITUNGEN ZUR AUFRECHTERHALTUNG UND/ODER WIEDERHERSTELLUNG GESUNDER HAUTMIKROBIOTA
PRÉPARATIONS DERMATOLOGIQUES DESTINÉES À MAINTENIR ET/OU RESTAURER UN MICROBIOTE CUTANÉ SAIN

(30) Priority: 21.06.2016 BE 201605454
(43) Date of publication of application: 24.04.2019
(73) Proprietor: YUN NV, 2630 Aartselaar (BE); Universiteit Antwerpen, 2000 Antwerpen (BE)
(72) Inventor: LEBEER, Sarah, 2020 Antwerpen (BE); CLAES, Ingmar, 2020 Antwerpen (BE); OERLEMANS, Eline, 2020 Antwerpen (BE); VAN DEN BROEK, Marianne, 2020 Antwerpen (BE)
(74) Representative: Arnold & Siedsma
(86) International application number: PCT/EP2017/065006
(87) International publication number: WO 2017/220525

(56) References cited:
- EP-A1- 1 736 537
- EP-A1- 2 149 368
- EP-A1- 2 332 521
- WO-A1-2016/023688
- WO-A2-01/13956
- WO-A2-2011/052996
- WO-A2-2013/188626
- JP-A- 2008 013 502
- JP-A- 2015 117 234
- KR-A- 20120 089 530
- KR-A- 20150 075 447

### SEQUENCE LISTING

<110> YUN NV
<120> DERMATOLOGICAL PREPARATIONS FOR MAINTAINING AND/OR RESTORING HEALTHY
   SKIN MICROBIOTA
<130> YUN-002
<150> BE2016/5454
   <151> 2016-06-21
<170> BiSSAP 1.3.6
<210> 1
   <211> 1406
   <212> RNA
   <213> Lactobacillus pentosus
   <223> 16S rRNA sequence
<400> 1
   cttaggcggc tggttcctaa aaggttaccc caccgacttt gggtgttaca aactctcatg      60
   gtgtgacggg cggtgtgtac aaggcccggg aacgtattca ccgcggcatg ctgatccgcg     120
   attactagcg attccgactt catgtaggcg agttgcagcc tacaatccga actgagaatg     180
   gctttaagag attagcttac tctcgcgagt tcgcaactcg ttgtaccatc cattgtagca     240
   cgtgtgtagc ccaggtcata aggggcatga tgatttgacg tcatccccac cttcctccgg     300
   tttgtcaccg gcagtctcac cagagtgccc aacttaatgc tggcaactga taataagggt     360
   tgcgctcgtt gcgggactta acccaacatc tcacgacacg agctgacgac aaccatgcac     420
   cacctgtatc catgtccccg aagggaacgt ctaatctctt agatttgcat agtatgtcaa     480
   gacctggtaa ggttcttcgc gtagcttcga attaaaccac atgctccacc gcttgtgcgg     540
   gcccccgtca attcctttga gtttcagcct tgcggccgta ctccccaggc ggaatgctta     600
   atgcgttagc tgcagcactg aagggcggaa accctccaac acttagcatt catcgtttac     660
   ggtatggact accagggtat ctaatcctgt ttgctaccca tactttcgag cctcagcgtc     720
   agttacagac cagacagccg ccttcgccac tggtgttctt ccatatatct acgcatttca     780
   ccgctacaca tggagttcca ctgtcctctt ctgcactcaa gtttcccagt ttccgatgca     840
   cttcttcggt tgagccgaag gctttcacat cagacttaaa aaaccgcctg cgctcgcttt     900
   acgcccaata aatccggaca acgcttgcca cctacgtatt accgcggctg ctggcacgta     960
   gttagccgtg gctttctggt taaataccgt caatacctga acagttactc tcagatatgt    1020
   tcttctttaa caacagagtt ttacgagccg aaacccttct tcactcacgc ggcgttgctc    1080
   catcagactt tcgtccattg tggaagattc cctactgctg cctcccgtag gagtttgggc    1140
   cgtgtctcag tcccaatgtg gccgattacc ctctcaggtc ggctacgtat cattgccatg    1200
   gtgagccgtt accccaccat ctagctaata cgccgcggga ccatccagaa gtgatagccg    1260
   aagccatctt tcaaactcgg accatgcggt ccaagttgtt atgcggtatt agcatctgtt    1320
   tccaggtgtt atcccccgct tctgggcagg tttcccacgt gttactcacc agttcgccac    1380
   tcactcaaat gtaaatcatg atgcaa                                         1406
<210> 2
   <211> 20
   <212> DNA
   <213> Artificial Sequence
   <223> Primer 8F
<400> 2
   agagtttgat cctggctcag 20
<210> 3
   <211> 20
   <212> DNA
   <213> Artificial Sequence
   <223> Primer 1525R
<400> 3
   aaggaggtga tccagccgca 20
<210> 4
   <211> 1425
   <212> RNA
   <213> Lactobacillus plantarum
   <223> 16S rRNA
<400> 4
   ggttcctaaa aggttacccc accgactttg ggtgttacaa actctcatgg tgtgacgggc      60
   ggtgtgtaca aggcccggga acgtattcac cgcggcatgc tgatccgcga ttactagcga     120
   ttccgacttc atgtaggcga gttgcagcct acaatccgaa ctgagaatgg ctttaagaga     180
   ttagcttact ctcgcgagtt cgcaactcgt tgtaccatcc attgtagcac gtgtgtagcc     240
   caggtcataa ggggcatgat gatttgacgt catccccacc ttcctccggt ttgtcaccgg     300
   cagtctcacc agagtgccca acttaatgct ggcaactgat aataagggtt gcgctcgttg     360
   cgggacttaa cccaacatct cacgacacga gctgacgaca accatgcacc acctgtatcc     420
   atgtccccga agggaacgtc taatctctta gatttgcata gtatgtcaag acctggtaag     480
   gttcttcgcg tagcttcgaa ttaaaccaca tgctccaccg cttgtgcggg cccccgtcaa     540
   ttcctttgag tttcagcctt gcggccgtac tccccaggcg gaatgcttaa tgcgttagct     600
   gcagcactga agggcggaaa ccctccaaca cttagcattc atcgtttacg gtatggacta     660
   ccagggtatc taatcctgtt tgctacccat actttcgagc ctcagcgtca gttacagacc     720
   agacagccgc cttcgccact ggtgttcttc catatatcta cgcatttcac cgctacacat     780
   ggagttccac tgtcctcttc tgcactcaag tttcccagtt tccgatgcac ttcttcggtt     840
   gagccgaagg ctttcacatc agacttaaaa aaccgcctgc gctcgcttta cgcccaataa     900
   atccggacaa cgcttgccac ctacgtatta ccgcggctgc tggcacgtag ttagccgtgg     960
   ctttctggtt aaataccgtc aatacctgaa cagttactct cagatatgtt cttctttaac    1020
   aacagagttt tacgagccga aacccttctt cactcacgcg gcgttgctcc atcagacttt    1080
   cgtccattgt ggaagattcc ctactgctgc ctcccgtagg agtttgggcc gtgtctcagt    1140
   cccaatgtgg ccgattaccc tctcaggtcg gctacgtatc attgccatgg tgagccgtta    1200
   ccccaccatc tagctaatac gccgcgggac catccaaaag tgatagccga agccatcttt    1260
   caagctcgga ccatgcggtc caagttgtta tgcggtatta gcatctgttt ccaggtgtta    1320
   tcccccgctt ctgggcaggt ttcccacgtg ttactcacca gttcgccact cactcaaatg    1380
   taaatcatga tgcaagcacc aatcaatacc agagttcgtt cgact                    1425
<210> 5
   <211> 1403
   <212> RNA
   <213> Lactobacillus rhamnosus
   <223> 16S rRNA
<400> 5
   gtcgacgagt tctgattatt gaaaggtgct tgcatcttga tttaattttg aacgagtggc      60
   ggacgggtga gtaacacgtg ggtaacctgc ccttaagtgg gggataacat ttggaaacag     120
   atgctaatac cgcataaatc caagaaccgc atggttcttg gctgaaagat ggcgtaagct     180
   atcgcttttg gatggacccg cggcgtatta gctagttggt gaggtaacgg ctcaccaagg     240
   caatgatacg tagccgaact gagaggttga tcggccacat tgggactgag acacggccca     300
   aactcctacg ggaggcagca gtagggaatc ttccacaatg gacgcaagtc tgatggagca     360
   acgccgcgtg agtgaagaag gctttcgggt cgtaaaactc tgttgttgga gaagaatggt     420
   cggcagagta actgttgtcg gcgtgacggt atccaaccag aaagccacgg ctaactacgt     480
   gccagcagcc gcggtaatac gtaggtggca agcgttatcc ggatttattg ggcgtaaagc     540
   gagcgcaggc ggttttttaa gtctgatgtg aaagccctcg gcttaaccga ggaagtgcat     600
   cggaaactgg gaaacttgag tgcagaagag gacagtggaa ctccatgtgt agcggtgaaa     660
   tgcgtagata tatggaagaa caccagtggc gaaggcggct gtctggtctg taactgacgc     720
   tgaggctcga aagcatgggt agcgaacagg attagatacc ctggtagtcc atgccgtaaa     780
   cgatgaatgc taggtgttgg agggtttccg cccttcagtg ccgcagctaa cgcattaagc     840
   attccgcctg gggagtacga ccgcaaggtt gaaactcaaa ggaattgacg ggggcccgca     900
   caagcggtgg agcatgtggt ttaattcgaa gcaacgcgaa gaaccttacc aggtcttgac     960
   atcttttgat cacctgagag atcaggtttc cccttcgggg gcaaaatgac aggtggtgca    1020
   tggttgtcgt cagctcgtgt cgtgagatgt tgggttaagt cccgcaacga gcgcaaccct    1080
   tatgactagt tgccagcatt tagttgggca ctctagtaag actgccggtg acaaaccgga    1140
   ggaaggtggg gatgacgtca aatcatcatg ccccttatga cctgggctac acacgtgcta    1200
   caatggatgg tacaacgagt tgcgagaccg cgaggtcaag ctaatctctt aaagccattc    1260
   tcagttcgga ctgtaggctg caactcgcct acacgaagtc ggaatcgcta gtaatcgcgg    1320
   atcagcacgc cgcggtgaat acgttcccgg gccttgtaca caccgcccgt cacaccatga    1380
   gagtttgtaa cacccgaagc cgg                                            1403
<210> 6
   <211> 19
   <212> DNA
   <213> Artificial Sequence
   <223> Primer Sequence
<400> 6
   tccgtaggtg aacctgcgg 19
<210> 7
   <211> 20
   <212> DNA
   <213> Artificial Sequence
   <223> Primer Sequence
<400> 7
   tcctccgctt attgatatgc 20

## Description

### FIELD OF THE INVENTION

The present invention is directed to the direct topical application of beneficial or probiotic bacteria to the skin for maintenance of a healthy skin microbiota and to help restore an unbalanced skin microbiota. This restoration of a healthy microbiota falls under the term probiotherapy, defined as the use of beneficial micro-organisms or probiotics to restore a healthy microbiota at a site where microbial dysbiosis occurs. The application is based on the use of selected *Lactobacillus* strains as anti-pathogenic agents, in particular *L. plantarum, L. pentosus* and/or *L. rhamnosus,* against common skin pathogens, whereby produced acids such as lactic acid are important antimicrobial factors.

### BACKGROUND TO THE INVENTION

Hence, it was an object of the present invention to provide a solution for subjects suffering from skin conditions due to an aberrant microbial balance on the skin. Thereto, it was found that the topical use of *L. plantarum,* L. *pentosus* and/or *L. rhamnosus* species on the skin is very effective in restoring and/or maintaining a healthy skin microbiota, and is thus very suitable in relieving skin conditions in subjects in need thereof.

Oral formulations comprising *Lactobacillus* strains have been used before in the treatment of skin conditions like atopic dermatitis. However, oral administration versus direct topical administration are different administration routes and each have a completely different underlying mechanism. In oral administration, in particular a beneficial effect on the general health via immuno-stimulation is intended, whereas by direct dermatological (skin) administration, competition with 'unwanted' microorganisms occurs.

Like the gastrointestinal tract, our skin harbours a unique microbial ecosystem. The type of micro-organisms found on the skin depends on a combination of host factors, environmental factors but also topographical location. The role of this microbiota in skin disorders is still not completely unravelled. However, it seems that, at least, some skin disorders are linked to a disturbed microbiota as antimicrobial treatments can improve clinical symptoms (Grice & Segre 2011). For example, in acne vulgaris a correlation has been found with the presence of *Propionibacterium acnes* (Beylot et al. 2014). Although acne vulgaris is a multifactorial condition and is, among other factors, influenced by hormonal factors, these *P. acnes* bacteria seem to induce inflammation resulting in inflamed pimples also called papules or pustels. As *P. acnes* is also found on a healthy skin not causing acne, this suggests that other factors are involved, tipping the balance of the composition of the skin microbiota towards an overgrowth of this bacteria.

Another example of a skin disorder where the microbiota seems to be important is dandruff (Wang et al. 2015; Sugita et al. 2015; Grice & Segre 2011). In people with dandruff, the fungus *Malassezia* is often overrepresented. Indications that it is this fungus that is a possible cause of the condition, come from the fact that antimycotic treatment improves the symptoms. In contrast, antibacterial therapies do not improve dandruff. Again, other factors are expected to be involved in this skin disorder but the correlation with *Malassezia* is intruiging.

Similarly as for dandruff, fungal skin infections with *Candida albicans* or dermatophytes, like *Trichophyton spp.,* seem to be skin disorders linked to a dysbiosis in the skin microbiota as these species are also present on healthy subjects. In the case of Tinea pedis or 'athlete's foot' overgrowth of *Trichophyton rubrum* or *T. mentagrophytes* is often observed.

The production of lactic acid in combination with possibly other antimicrobial compounds like bacteriocins seems to give protection against aforementioned infections and dysbiotic conditions and lactic acid seems to be active against bacterial, fungal and even viral pathogens. It is for this reason that lactobacilli are considered to be important in the homeostasis of the dynamical dermatological ecosystem. Potential health promoting mechanisms of lactobacilli are i) to preserve a healthy skin pH (+/- 5.5), mainly by production of lactic acid; ii) production of antimicrobial compounds and competitive exclusion of pathogens; iii) modulation of immune response and iv) strengthening of the epithelial barrier.

Hence, it was an object of the present invention to provide a solution for subjects suffering from dermatological conditions due to an aberrant microbial balance of the skin. Thereto, it was found that the topical dermatological use of L. *plantarum,* L. *pentosus* and/or L. rhamnosus species is very effective in restoring and/or maintaining a healthy microbiota on the skin, and is thus very suitable in relieving dermatological conditions in subjects in need thereof.

Oral formulations comprising *Lactobacillus* strains have been used before in the treatment of dermatological disorders. However, oral administration versus direct topical administration are different administration routes and each have a completely different underlying mechanism. In oral administration, in particular a beneficial effect on the general health via immuno-stimulation is intended, whereas by direct administration on the skin, competition with 'unwanted' microorganisms occur. WO0113956 discloses compositions for topical administration. In the following, the references to methods of treatment in the subsequent paragraphs of this description are to be interpreted as references to the compounds, pharmaceutical compositions and medicaments of the present invention for use in a method for treatment of the human (or animal) body by therapy.

### SUMMARY OF THE INVENTION

The scope of the invention is defined by the appended claims. In a first aspect, the present invention provides a topical dermatological composition comprising live *Lactobacillus* species *L. plantarum, L. pentosus* and *L. rhamnosus;* wherein said *L. plantarum* is a *L. plantarum* strain having at least 97% sequence similarity with SEQ ID N° 4 in its 16S rRNA gene, said *L. pentosus* is a *L. pentosus* strain having at least 97% sequence similarity with SEQ ID N° 1 in its 16S rRNA gene, and said *L. rhamnosus* is a *L. rhamnosus* strain having at least 97% sequence similarity with SEQ ID N° 5 in its 16S rRNA gene.

In a further aspect, the present invention provides said composition that is suitable for use in restoring and/or maintaining a healthy skin microbiota, by topical route..

Disclosed herein is the non-therapeutic use of said topical dermatological composition for restoring and/or maintaining a healthy skin microbiota.

Also disclosed herein is said non-therapeutic use of said topical dermatological composition or said topical dermatological composition, wherein said composition is a topical skin composition in the form of a gel, cream, ovule, suppository forms, foam, lotion, or ointment.

Further disclosed herein is said non-therapeutic use of said topical dermatological composition, wherein said *Lactobacillus* species are *L. plantarum* YUN-V2.0 deposited under accession number LMG P-29456 (deposited at BCCM on Mar, 09 2016), *L. pentosus* YUN-V1.0 deposited under accession number LMG P-29455 (deposited at BCCM on Mar, 09 2016); and L. rhamnosus YUN-S1.0 deposited under accession number LMG P-29611 (deposited at BCCM on May, 12 2016).

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

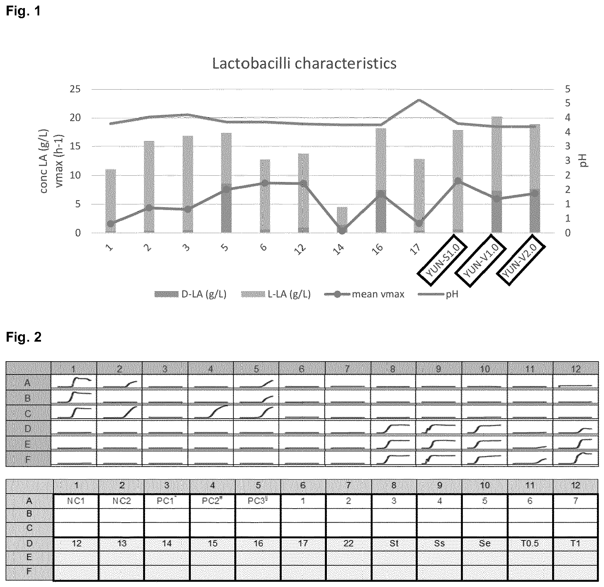

**Fig. 1****:** Characteristics of lactobacilli in reference to growth, production of D- and L-lactic acid (LA) and lowering of the pH of the medium.
**Fig. 2****:** Time course experiment for the analysis of the antipathogenic effect of spent culture supernatant of lactobacilli against *Propionibacterium acnes.* Growth of the bacteria (optical density at 600nm; Y-axis) is measured in time (X-axis). Each graph shows replicates of growth of *P. acnes.* It can be clearly noted that without any addition of antibiotic or SCS, *P. acnes* quickly starts to grow (NC1). Similar as when erythromycin at 50µg/ml is added, SCS of all lactobacilli prevents growth of *P. acnes* while SCS of streptococci or staphylococci does not inhibit growth. *Erythromycine (50ug/ml); ^{#}Erythromycine (5ug/ml); ^{§}Minocycline (20 µg/ml) NC1=medium control; NC2=MRS at pH4.3; Numbers 1 to 22 = lactobacilli strains (for details see table 1); St=*Streptococcus thermophilus;* Ss=*Streptococcus salivarius;* Se=*Staphylococcus epidermidis;* T0.5=0.5% Tween 80; T1=1% Tween 80.

### DETAILED DESCRIPTION OF THE INVENTION

The present invention is based on the discovery of specific *Lactobacillus* strains that can compete with growth of *Propionibacterium acnes, Candida albicans, Malassezia spp., Trichophyton spp.* and bacteria or fungi that are linked with skin conditions like acne vulgaris, dandruff, tinea pedis or other fungal skin infections. These selected strains are herein generally termed "YUN" strains and are capable of competing with skin pathogens and thereby restore a healthy skin microbiota. This restoration of a healthy microbiota falls under the term probiotherapy, defined as the use of beneficial micro-organisms or probiotics to restore a healthy microbiota at a site where microbial dysbiosis occurs.

Hence, in a first aspect, the present invention provides a topical dermatological composition comprising live *Lactobacillus* species *L. plantarum, L. pentosus* and *L. rhamnosus;* wherein said *L. plantarum* is a *L. plantarum* strain having at least 97% sequence similarity with SEQ ID N° 4 in its 16S rRNA gene, said *L. pentosus* is a *L. pentosus* strain having at least 97% sequence similarity with SEQ ID N° 1 in its 16S rRNA gene, and said *L. rhamnosus* is a *L. rhamnosus* strain having at least 97% sequence similarity with SEQ ID N° 5 in its 16S rRNA gene.

Also disclosed herein is that said composition further comprises *Lactobacillus* species such as for example selected from the non-limiting list comprising *L. pentosus, L. gasseri, L. crispatus, L. acidophilus, L. jensenii, L. fermentum, L. rhamnosus.*

In the context of the present invention, the term "topical" is meant to be the local delivery at a specified location of the body, in particular the application to a particular place on the body. In particular, it includes the application via non-solid formulations such as creams, foams, gels, lotions or ointments. The term "topical" is not meant to include the delivery in the form of solid preparations such as capsules, tablets, ...

Hence, the term "topical skin" is meant to include the local delivery using non-solid formulations directly onto the skin of the body. Preferably, the compositions according to the present invention are applied over a large area of the skin in order to be most effective.

In the context of the present invention the term "live *Lactobacillus* species" is meant to be viable *Lactobacillus* species, and is not meant to be fragments, culture supernatants, or killed forms thereof.

In a further aspect, the present invention provides said topical dermatological composition for use in restoring and/or maintaining a healthy skin microbiota, by topical route

Also disclosed herein is said topical dermatological composition for use in probiotherapy of the skin, by topical route. As already defined herein above, said probiotherapy is meant to be the restoration and/or maintainance of a healthy skin microbiota in a subject in need thereof.

Subjects that may benefit from such probiotherapy are for example people/persons with a skin conditions linked to a disturbed skin microbiota possibly due to bacterial or yeast infections and/or any dysbiosis caused by overgrowth of specific pathogenic micro-organisms, like acne vulgaris, tinea pedis, dandruff, rosaceae, impetigo,...

Therefore, disclosed herein is the non-therapeutic use of said topical dermatological composition for restoring and/or maintaining a healthy skin microbiota.

Furthermore, disclosed herein is said non-therapeutic use of said topical dermatological composition wherein said composition is a topical skin composition in the form of a gel, cream, ovule, suppository forms, foam, lotion, or ointment.

In a further aspect the invention provides the topical dermatological composition, wherein said composition is a topical skin composition in the form of a gel, cream, ovule, suppository forms, foam, lotion, or ointment.

Further disclosed herein is a method for restoring and/or maintaining a healthy skin microbiota; comprising at least one step of administering by topical route, to an individual, an effective amount of one or more live *Lactobacillus* species; wherein at least one of said *Lactobacillus* species is *L. plantarum*; more in particular a *L. plantarum* strain having at least 97% sequence similarity with SEQ ID N° 4 in its 16S rRNA gene.

Additionally is disclosed herein a composition comprising one or more live *Lactobacillus* species for use in restoring and/or maintaining a healthy skin microbiota, by topical route, said *Lactobacillus* species being selected from the list comprising L. *plantarum, L. pentosus* and *L. rhamnosus*; more in particular a L. *plantarum* strain having at least 97% sequence similarity with SEQ ID N° 4 in its 16S rRNA gene; a *L. pentosus* strain having at least 97% sequence similarity with SEQ ID N° 1 in its 16S rRNA gene and a L. rhamnosus strain having at least 97% sequence similarity with SEQ ID N° 5 in its 16S rRNA gene.

Further, disclosed herein is said non-therapeutic use of said topical dermatological composition, wherein said *Lactobacillus* species are *L. pentosus* YUN-V1.0 deposited under accession number LMG P-29455 (deposited at BCCM on Mar, 09 2016); *L. plantarum* YUN-V2.0 deposited under accession number LMG P-29456 (deposited at BCCM on Mar, 09 2016); and L. rhamnosus YUN-S1.0 deposited under accession number LMG P-29611 (deposited at BCCM on May, 12 2016).

The present invention further provides the topical dermatological composition, wherein said *Lactobacillus* species are *L. pentosus* YUN-V1.0 deposited under accession number LMG P-29455 (deposited at BCCM on Mar, 09 2016); *L. plantarum* YUN-V2.0 deposited under accession number LMG P-29456 (deposited at BCCM on Mar, 09 2016); and L. rhamnosus YUN-S1.0 deposited under accession number LMG P-29611 (deposited at BCCM on May, 12 2016)

The microbiological deposits mentioned herein, have been made with the BCCM/LMG Bacteria collection ("Belgian co-ordinated collections of micro-organism") with correspondence address: Laboratorium voor Microbiologie, Universiteit Gent, K.L. Ledeganckstraat 35 - 9000 Gent, Belgium

*Lactobacillus pentosus* YUN-V1.0 is a single colony isolate obtained in our lab after subculturing of a strain, that was originally a vaginal isolate of healthy woman. The 16S rRNA gene sequence (SEQ ID N° 1) for strain *L. pentosus* YUN-V1.0 was determined by PCR using primers 8F (5'-AGAGTTTGATCCTGGCTCAG-3' - SEQ ID N° 2) and 1525R (5'-AAGGAGGTGATCCAGCCGCA-3' - SEQ ID N° 3).

YUN-V2.0 and YUN-V3.0 are single colony isolates obtained in our lab after subculturing of *Lactobacillus plantarum* strains that were originally isolated from human saliva and a maize silage respectively. The 16S rRNA gene sequence (SEQ ID N° 4) for strain *L. plantarum* YUN-V2.0 was determined by PCR using primers 8F (5'-AGAGTTTGATCCTGGCTCAG-3' - SEQ ID N° 2) and 1525R (5'-AAGGAGGTGATCCAGCCGCA-3' - SEQ ID N° 3).

YUN-S1.0 is a single colony isolate obtained in our lab after subculturing of a *Lactobacillus rhamnosus* strain that was originally isolated from a healthy person. The 16S rRNA gene sequence (SEQ ID N° 5) for strain *L. rhamnosus* YUN-S1.0 was determined by PCR using primers 8F (5'-AGAGTTTGATCCTGGCTCAG-3' - SEQ ID N° 2) and 1525R (5'-AAGGAGGTGATCCAGCCGCA-3' - SEQ ID N° 3).

These particular "YUN" strains can either be used as such, or are preferably formulated in a composition comprising such strains. Said compositions are topical skin compositions more in particular in the form of non-solid formulations such as creams, foams, gels, lotions or ointments.

In particular, also disclosed herein are the above defined "YUN" strains for use in probiotherapy of the skin, i.e. for restoring and/or maintaining a healthy skin microbiota.

Furthermore disclosed herein is a topical use of one or more live *Lactobacillus* species in probiotherapy of the skin; wherein said *Lactobacillus* species are selected from the list comprising *L. plantarum, L. pentosus* and *L. rhamnosus;* more in particular, said probiotherapy consists of restoring and/or maintaining a healthy skin microbiota in a subject in need thereof.

In a specific embodiment, the *Lactobacillus* species in the topical uses, methods and compositions as disclosed herein, is a *Lactobacillus* strain selected from the list comprising *L. plantarum* YUN-V2.0 deposited under accession number LMG P-29456 (deposited at BCCM on Mar, 09 2016); *L. pentosus* YUN-V1.0 deposited under accession number LMG P-29455 (deposited at BCCM on Mar, 09 2016); and *L. rhamnosus* YUN-S1.0 deposited under accession number LMG P-29611 (deposited at BCCM on May, 12 2016).

### EXAMPLES

### MATERIALS AND METHODS

### Bacterial strains and growth conditions

Lactobacillus strains (Table 1) were grown at 37°C in de Man, Rogosa and Sharpe (MRS) medium (Carl Roth). All bacteria were grown in non-shaking conditions and inoculated from glycerol stocks (-80°C). Solid media contained 1.5% (w/v) agar.

**Table 1: Bacterial strains used in this research**

| **Species** | **#** | **Strain** | **Relevant genotype or description** | **Reference and/or Source** |
|---|---|---|---|---|
| **LACTOBACILLI** | | | | |
| ***Lactobacillus casei*** | 1 | ATCC334 | Single colony isolate obtained in our lab from a stock culture of ATCC334 | ATCC |
| ***Lactobacillus casei*** | 2 | DN-114001 | Single colony isolate obtained in our lab from a commercially available fermented drink (Actimel^{®}) containing *L. casei* DN-114001, confirmed by sequencing | Commercial probiotic product |
| ***Lactobacillus casei*** | 3 | Shirota | Single colony isolate obtained in our lab from a commercially available fermented drink containing *L. casei* Shirota (Yakult^{®}), confirmed by sequencing | Commercial probiotic product |
| ***Lactobacillus pentosus*** | 4 | YUN-V1.0 | Single colony isolate | |
| ***Lactobacillus plantarum*** | 5 | LMG1284 | Single colony isolate from *L. plantarum* ATCC8014 or LMG1284 | ATCC |
| ***Lactobacillus reuteri*** | 6 | RC-14 | Single colony isolate obtained in our lab from a commercially available probiotic supplement containing *L. reuteri* RC-14, confirmed by sequencing | Commercial probiotic product |
| ***Lactobacillus rhamnosus*** | 7 | YUN-S1.0 | Clinical isolate | |
| ***Lactobacillus rhamnosus*** | 12 | GR-1 | Single colony isolate obtained in our lab from a commercially available probiotic supplement containing *L. rhamnosus* GR-1 | (Chan et al. 1984; 1985; Reid 1999; Reid & Bruce 2001), ATCC |
| ***Lactobacillus helveticus*** | 14 | AM B-2 | single colony isolate | Commercial probiotic product |
| ***Lactobacillus plantarum*** | 15 | YUN-V2.0 | Single colony isolate | |
| ***Lactobacillus plantarum*** | 16 | 5057 | Single colony isolate | |
| ***Lactobacillus paracasei*** | 17 | LMG12586 | Single colony isolate obtained in our lab from a stock culture of LMG12586 | BCCM/LMG |
| ***Lactobacillus plantarum*** | 22 | / | Single colony isolate | |
| ***Lactobacillus pentosus*** | 25 | LMG8041 | Single colony isolate | BCCM/LMG |

| **PATHOGENS** | | | | |
|---|---|---|---|---|
| ***Trichophyton rubrum*** | 2 | / | Clinical isolate | BCCM/LMG |
| ***Malassezia furtur*** | | / | Clinical isolate | BCCM/LMG |
| ***Candida albicans*** | / | / | Clinical isolate | |

### Preparation of spent culture supernatant (SCS) of selected strains

To obtain spent culture supernatant (SCS) containing the secreted active antimicrobial products, growth medium specific for each species was inoculated from a preculture and incubated for 24h. SCS was obtained by centrifugation for 30 min. at 6797 g (8000 rpm) at 4°C. Afterwards, the SCS was filter sterilized (0.20 µm cellulose acetate, VWR).

### Antimicrobial activity assays for co-cultures of live lactobacilli against Malassezia furfur, Trichophyton rubrum, Propionibacterium acnes and Candida albican.

The antimicrobial activity of the selected bacteria was explored by standard antimicrobial tests with some minor modifications. The antimicrobial activity of the selected bacteria was explored by spot assay (Schillinger and Lücke 1989). Briefly, 1-3 µL of each culture was spotted on an agar plate. These plates were incubated for 24h up to 72h depending on the strain. Next, an overnight culture of the pathogen was diluted into 7 mL of soft agar of the medium of the pathogen and poured over the plates with the spots of the selected strains. The plates were incubated overnight at 30-37°C, after which the inhibition zones were measured. A spot of miconazole (for fungi) and/or 0.1% hexetidine and/or tetracycline (for Propionibacterium acnes) was added to the spot plate as positive control before the soft agar was poured.

### Radial diffusion test of SCS of lactobacilli

In addition, the antimicrobial activity of spent culture supernatant (SCS) was investigated with a protocol as previously described for the competition assays between lactobacilli and gastrointestinal pathogens (Coconnier et al. 1997). Miconazol (for fungi) and tetracycline (for Propionibacterium acnes) was used as a positive control. Sterile growth medium was used as a negative control.

### Time course analysis of the antimicrobial activity of SCS of the selected strains against Candida, Propionibacterium acnes, Malassezia furfur and Trichophyton spp (further referred to as 'pathogens').

The time course analysis was performed similarly as described previously (De Keersmaecker et al. 2006) with minor modifications. Briefly, an overnight culture of the pathogen was added to the wells of a microplate filled with 50-80% the proper medium supplemented with 50-5% SCS of lactobacilli. MRS at pH 4,3 and antibiotics or antimycotics at the proper concentration were used as a negative and a positive control, respectively. Bacteria or fungi were grown and the optical density (OD) was measured at 590 nm each 30 min during 3 days using a Synergy HTX multimode reader (Biotek). Each test was measured at least in triplicate and the average OD was calculated. The antimicrobial activity was expressed as the relative optical density reached after 24h (stationary phase) compared to the negative controls.

### Antibiotic susceptibility

Antibiotic sensitivity was evaluated using the Kirby-Bauer disc diffusion test. In short, antibiotics were spotted on paper discs and the bacterial inhibition zone was measured on agar plates. The antibiotics tested were erythromycin, normocin, tetracyclin, ampicillin and clindamycin at relevant concentrations.

### Proof of concept human clinical trial in patients with acne vulgaris

A proof-of-concept clinical trial was performed on 20 patients with acne vulgaris. Patients were men between 12-25 years with mild inflammatory acne. The aim of this proof-of-concept trial was to assess the impact of a topical probiotic cream (containing +-10-8 colony forming units (CFU) of L. pentosus YUN-V1.0, +-10-8 CFU of L. plantarum YUN-V2.0 and +-10-8 CFU L. rhamnosus YUN-S1.0 per application of 1g of the topical cream ACN) on the skin microbiota and on the acne severity. Patients were asked to apply the cream twice daily for 56 days (8 weeks). The patients were seen by a dermatologist at start (before the therapy), week 4, week 8 and week 10. A skin swab was taken at each visit. Bacterial DNA was isolated from these samples by the commercial MoBio Powersoil kit (cfr. Human Microbiome Project). Isolated DNA was analysed via 16S rRNA amplicon sequencing with MiSeq Illumina and a bio-informatical analysis was performed. Moreover, a clinical scoring was performed and a photograph taken at each visit.

### Proof of concept human clinical trial in patients with tinea pedis (Athlete's foot)

A proof-of-concept clinical trial was performed on 20 patients with tinea pedis. Patients were between 18-65 years having tinea pedis. The aim of this proof-of-concept trial was to assess the impact of a topical probiotic cream (containing +-10-8 colony forming units (CFU) of L. pentosus YUN-V1.0, +-10-8 CFU of L. plantarum YUN-V2.0 and +-10-8 CFU L. rhamnosus YUN-S1.0 per application of 1g of of the topical cream FNG) on the skin microbiota and on the Trichophyton infection. Patients were asked to apply the cream twice daily for 56 days (8 weeks). The patients were seen by a dermatologist at start (before the therapy), week 4, week 8 and week 10. A skin swab was taken at each visit. Bacterial DNA was isolated from these samples by the commercial MoBio Powersoil kit (cfr. Human Microbiome Project). Isolated DNA was analysed via 16S rRNA amplicon sequencing with MiSeq Illumina and a bio-informatical analysis was performed. For analysis of the presence of the fungi, swabs were also plated out on Trichophyton specific medium (medium suggested by BCCM). Colony PCR using universal ITS (`internal transcribed region') primers ITS1 (SEQ ID N°6) (5'-TCCGTAGGTGAACCTGCGG-3') and ITS4 (SEQ ID N° 7) (5'-TCCTCCGCTTATTGATATGC-3') followed by sequencing was performed to identify the fungi. Moreover, a clinical scoring was performed and a photograph taken at each visit.

### RESULTS

### Growth characteristics and lactate production

Possible beneficial or probiotic strains were characterized in terms of growth characteristics, lactate production and ability of lowering of the pH of the medium. These characteristics are expected to be important for the antipathogenic activity. These data show that *Lactobacillus pentosus* YUN-V1.0 and *L. plantarum* YUN-V2.0 and *L. rhamnosus* YUN-S1.0 produce the highest amount of lactic acid (Fig 1).

### Antipathogenic activity against Propionibacterium acnes

Time course experiments were performed analyzing the antimicrobial activity of spent culture supernatant (SCS) of the selected strains against *Propionibacterium acnes.* SCS of all tested strains inhibited the growth of *Propionibacterium acnes* while SCS of other bacterial species like *Streptococcus thermophilus* and *S*. *salivarius,* both also lactic acid bacteria, and *Staphyloccus epidermidis* did not inhibit growth of *P*. *acnes.* This suggests species and perhaps strain specific properties of the selected lactobacilli to be important for the antipathogenic activity against *P*. *acnes* (Fig 2).

### Antipathogenic activity against Malassezia, Trichophyton and Candida

In a next phase, the beneficial or probiotic bacteria were screened for their antipathogenic effect against specific skin pathogens. The results of a spot assays against *Malassezia furfur, Trichophyton rubrum* and *Candida albicans* are shown in table 1, 2 and 3 respectively.

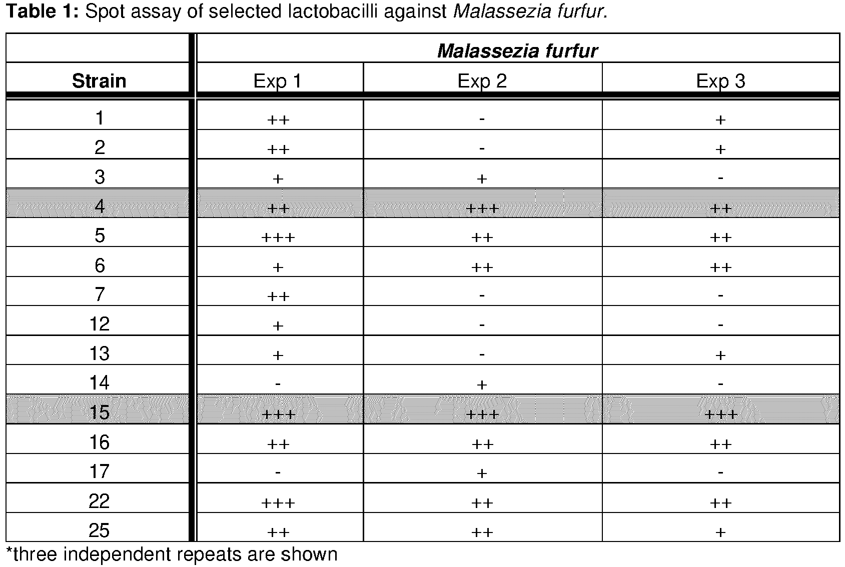

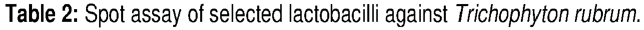

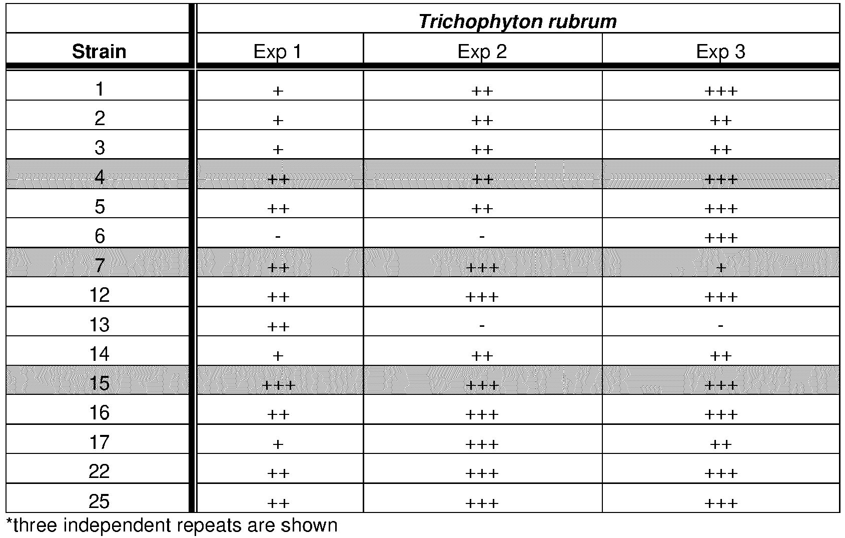

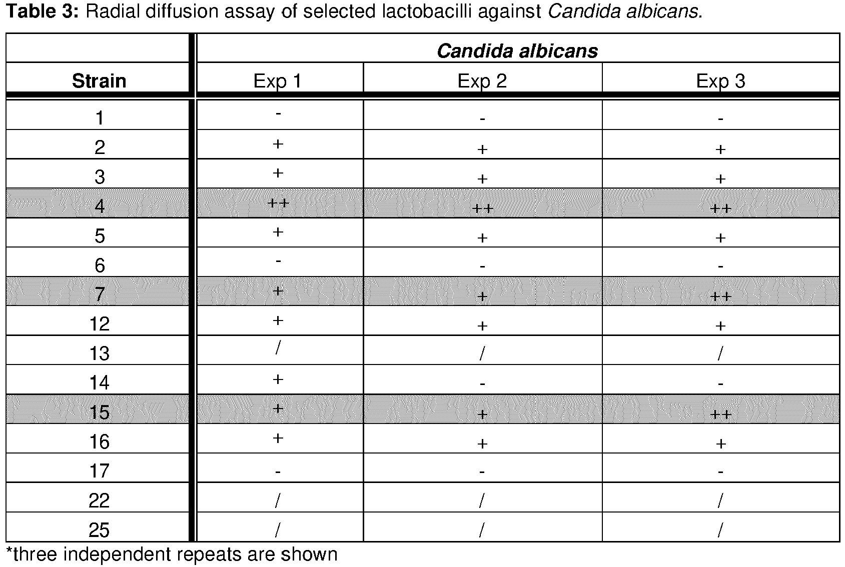

Spent culture supernatant from *L. pentosus* YUN-V1.0 and *L. plantarum* YUN-V2.0 was also tested in radial diffusion assays and demonstrated to be efficient in inhibiting *Malassezia, Trichophyton* and *Candida* growth. *L. rhamnosus* YUN-S1.0 was not as efficient in inhibiting growth of *Malassizia* but was able to inhibit growth of *Trichophyton* and *Candida.*

### Antibiotic susceptibility

The selected bacteria were also tested for their antibiotic susceptibility as to prevent spreading of antibiotic resistance genes. All lactobacilli were susceptible to erythromycin, normocin, tetracyclin, ampicillin and clindamycin, except for *L. plantarum* 5057, which was susceptible to tetracyclin. For this reason, strain *L. plantarum* 5057 was found not to be suitable to use as a strain for probiotherapy.

### REFERENCES

Beylot, C. et al., 2014. Propionibacterium acnes: an update on its role in the pathogenesis of acne. Journal of the European Academy of Dermatology and Venereology: JEADV, 28(3), pp.271-8.
Chan, R.C. et al., 1985. Competitive exclusion of uropathogens from human uroepithelial cells by Lactobacillus whole cells and cell wall fragments. Infection and immunity, 47(1), pp.84-9.
Chan, R.C., Bruce, A.W. & Reid, G., 1984. Adherence of cervical, vaginal and distal urethral normal microbial flora to human uroepithelial cells and the inhibition of adherence of gram-negative uropathogens by competitive exclusion. The Journal of urology, 131(3), pp.596-601.
Grice, E.A. & Segre, J.A., 2011. The skin microbiome. Nature reviews. Microbiology, 9(4), pp.244-53.
Reid, G., 1999. The Scientific Basis for Probiotic Strains of Lactobacillus. Appl. Envir. Microbiol., 65(9), pp.3763-3766.
Reid, G. & Bruce, A.W., 2001. Selection of lactobacillus strains for urogenital probiotic applications. The Journal of infectious diseases, 183 Suppl , pp.S77-80.
Sugita, T. et al., 2015. Temporal changes in the skin Malassezia microbiota of members of the Japanese Antarctic Research Expedition (JARE): A case study in Antarctica as a pseudo-space environment. Medical mycology, 53(7), pp.717-24.
Wang, L. et al., 2015. Characterization of the major bacterial-fungal populations colonizing dandruff scalps in Shanghai, China, shows microbial disequilibrium. Experimental dermatology, 24(5), pp.398-400.

## Claims

1. A topical dermatological composition comprising live *Lactobacillus* species *L. plantarum, L. pentosus* and *L. rhamnosus;* wherein said *L. plantarum* is a *L. plantarum* strain having at least 97% sequence similarity with SEQ ID N° 4 in its 16S rRNA gene, said *L. pentosus* is a *L. pentosus* strain having at least 97% sequence similarity with SEQ ID N° 1 in its 16S rRNA gene, and said *L. rhamnosus* is a *L. rhamnosus* strain having at least 97% sequence similarity with SEQ ID N° 5 in its 16S rRNA gene.

2. Composition as claimed in claim 1 for use in restoring and/or maintaining a healthy skin microbiota, by topical route.

3. The composition as claimed in claim 1 or the composition for use according to claim 2, wherein said composition is a topical skin composition in the form of a gel, cream, ovule, suppository forms, foam, lotion, or ointment.

4. The composition as claimed in claim 1 the composition for use according to claim 2, wherein said *Lactobacillus* species are *Lactobacillus* strains *L. plantarum* YUN-V2.0 deposited under accession number LMG P-29456 in combination with *L. pentosus* YUN-V1 .0 deposited under accession number LMG P-29455 and/or *L. rhamnosus* YUN-S1 .0 deposited under accession number LMG P-29611.

## Patentansprüche

1. Topische dermatologische Zusammensetzung, umfassend lebende *Lactobacillus-*Arten *L. plantarum, L. pentosus* und *L. rhamnosus;* wobei der *L.-plantarum-*Stamm ein *L.-plantarum-*Stamm ist, der mindestens 97 % Sequenzähnlichkeit mit SEQ ID N° 4 in seinem 16S-rRNA-Gen aufweist, der *L.-pentosus*-Stamm ein *L.-pentosus-*Stamm ist, der mindestens 97 % Sequenzähnlichkeit mit SEQ ID N° 1 in seinem 16S-rRNA-Gen aufweist, und der *L.-rhamnosus-*Stamm ein *L.-rhamnosus-*Stamm ist, der mindestens 97 % Sequenzähnlichkeit mit SEQ ID N° 5 in seinem 16S-rRNA-Gen aufweist.

2. Zusammensetzung nach Anspruch 1 zur Verwendung bei einem Wiederherstellen und/oder Aufrechterhalten einer gesunden Hautmikroflora auf topischem Wege.

3. Zusammensetzung nach Anspruch 1 oder Zusammensetzung zur Verwendung nach Anspruch 2, wobei die Zusammensetzung eine topische Hautzusammensetzung in Form eines Gels, einer Creme, einer Ovula, von Zäpfchenformen, eines Schaums, einer Lotion oder einer Salbe ist.

4. Zusammensetzung nach Anspruch 1, Zusammensetzung zur Verwendung nach Anspruch 2, wobei die *Lactobacillus-*Arten die *Lactobacillus-*Stämme *L. plantarum* YUN-V2.0, hinterlegt unter der Zugangsnummer LMG P-29456, in Kombination mit *L. pentosus* YUN-V1 .0, hinterlegt unter der Zugangsnummer LMG P-29455, und/oder *L. rhamnosus* YUN-S1 .0, hinterlegt unter der Zugangsnummer LMG P-29611, sind.

## Revendications

1. Composition dermatologique topique comprenant des espèces vivantes de *Lactobacillus* comme *L. plantarum, L. pentosus* et *L. rhamnosus* ; dans laquelle ledit *L*. *plantarum* est une souche de *L. plantarum* ayant au moins 97 % de similarité de séquence avec SEQ ID N° 4 dans son gène ARNr 16S, ledit *L. pentosus* est une souche de *L. pentosus* ayant au moins 97 % de similarité de séquence avec SEQ ID N° 1 dans son gène ARNr 16S, et ledit *L. rhamnosus* est une souche de *L. rhamnosus* ayant au moins 97 % de similarité de séquence avec SEQ ID N° 5 dans son gène ARNr 16S.

2. Composition selon la revendication 1 pour l'utilisation dans la restauration et/ou le maintien d'un microbiote cutané sain, par voie topique.

3. Composition selon la revendication 1 ou composition pour utilisation selon la revendication 2, dans laquelle ladite composition est une composition cutanée topique sous forme de gel, de crème, d'ovule, de formes suppositoires, de mousse, de lotion ou de pommade.

4. Composition selon la revendication 1, composition pour utilisation selon la revendication 2, dans laquelle lesdites espèces de *Lactobacillus* sont des souches de *Lactobacillus L. plantarum* YUN-V2.0 déposées sous le numéro d'enregistrement LMG P-29456 en combinaison avec *L. pentosus* YUN-V1 .0 déposé sous le numéro d'enregistrement LMG P-29455 et/ou *L. rhamnosus* YUN-S1 .0 déposé sous le numéro d'enregistrement LMG P-29611.
